# EUROPÄISCHE PATENTSCHRIFT

(11) **EP 0 678 513 B1**
(45) Veröffentlichungstag und Bekanntmachung des Hinweises auf die Patenterteilung: **16.02.2000**
(21) Anmeldenummer: 95105703.3
(22) Anmeldetag: 15.04.1995
(51) Int. Cl.: C07D 301/00, C07D 303/02, C07D 303/24, C08G 59/14

(54) **Verfahren zur selektiven Hydrierung von aromatischen Gruppen in Gegenwart von Epoxygruppen**
Process for the selective hydrogenation of aromatic groups in presence of epoxy groups
Procédé pour l'hydrogénation sélective de groupes aromatiques en présence de groupes époxy

(30) Priorität: 22.04.1994 DE 4414090
(43) Veröffentlichungstag der Anmeldung: 25.10.1995
(73) Patentinhaber: BASF Aktiengesellschaft, 67063 Ludwigshafen (DE)
(72) Erfinder: Wettling, Thomas, Dr., D-67117 Limburgerhof (DE); Schuster, Ludwig, Dr., D-67117 Limburgerhof (DE); Henkelmann, Jochem, Dr., D-68165 Mannheim (DE)

(56) Entgegenhaltungen:
- EP-A- 0 258 789
- EP-A- 0 402 743
- EP-A- 0 545 154
- FR-A- 2 516 916
- US-A- 3 336 241
- ACCOUNTS OF CHEMICAL RESEARCH, Bd.12, Nr.9, September 1979, WSAHINGTON DC US Seiten 324 - 331 E.L. MUTTERTIES ET AL. 'Catalytic hydrogenation of aromatic hydrocarbons'
- TETRAHEDRON LETTERS, Bd.36, Nr.6, 6. Februar 1995, OXFORD GB Seiten 885 - 888 F. FACHE ET AL. 'A catalytic stereo- and chemo-selective method for the reduction of aromatics'

## Beschreibung

Die vorliegende Erfindung betrifft ein verbessertes Verfahren zur selektiven Hydrierung von aromatischen Gruppen organischer Moleküle, die mindestens eine aromatische Gruppe und eine Epoxygruppe tragen, mit Wasserstoff in Gegenwart eines rutheniumhaltigen Katalysators.

Die US-A 3 336 241 lehrt die Hydrierung von aromatischen Epoxyverbindungen mit heterogenen Rhodium- und Rutheniumkatalysatoren. Die Selektivität dieser Katalysatoren ist nicht befriedigend, da bei der Hydrierung der aromatischen Gruppen in beträchtlichem Maße auch Epoxygruppen mit Wasserstoff reagieren.

Wesentlich selektiver als Katalysator ist Rutheniumoxidhydrat, wie es in der DE-A 36 29 632 und DE-A 39 19 228 für die Hydrierung von Di-[glycidoxi-phenyl]-methan bzw. 2,2-Di-[glycidoxi-phenyl]-propan beschrieben wird.

Da dieser Katalysator jedoch üblicherweise in Form einer lösungsmittelfeuchten Paste eingesetzt wird, bereitet seine genaue Dosierung technische Probleme.

Es bestand daher die Aufgabe, ein Verfahren mit solchen Katalysatoren bereitzustellen, welche einerseits eine möglichst selektive Hydrierung der aromatischen Gruppen ermöglichen und andererseits leicht dosierbar sein sollen. Eine weitere Aufgabe bestand darin, solche Katalysatoren zu finden, die in einfacher Weise heistellbar sind.

Demgemäß wurde das oben definierte Verfahren gefunden, das dadurch gekennzeichnet ist, daß man einen homogenen Rutheniumkatalysator verwendet, der durch Reduktion einer Rutheniumverbindung mit einem Metall, das ein Redoxpotential von -0,75 bis 2,5 V besitzt, einem Borhydrid, einem Aluminiumhydrid, einer Aluminiumalkylverbindung, eine Lithiumalkyl oder -arylverbindung in Gegenwart eines Ethers erhältlich ist.

Als Ausgangsverbindungen kommen alle solche organische Moleküle in Betracht, die mindestens eine aromatische Gruppe und eine Epoxygruppe tragen. Dabei kann es sich um monomere, oligomere oder polymere Verbindungen handeln. Als Ausgangsverbindungen für

Die EP-A-545 154 beschreibt ein Verfahren zur Herstellung von Diglycidyloligoethern durch katalytische Hydrierung, wobei man Rutheniumoxidhydrat als Katalysator verwendet und die Hydrierung bei 20 bis 60°C und einem Wasserstoffdruck von 100 bis 400 bar vornimmt. das erfindungsgemäße Verfahren sind folgende Substanzklassen und Stoffe zu nennen:
- Reaktionsprodukte aus Bisphenol A bzw. Bisphenol F und Epichlorhydrin
   Bisphenol A bzw. Bisphenol F und Epichlorhydrin können mit Basen in bekannter Weise (z.B. Ullmann's Encyclopedia of Industrial Chemistry, 5th Edition, VCH (1987) Vol. A9) zu Glycidylethern der allgemeinen Formel I umgesetzt werden 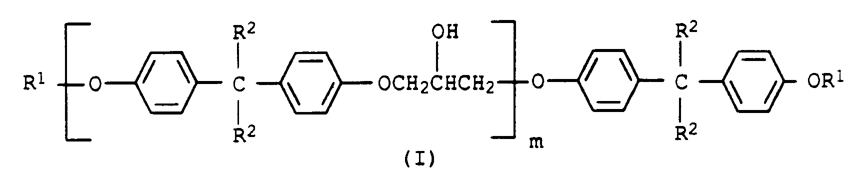 wobei R¹ für 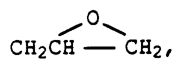 R² für Wasserstoff oder eine Methylgruppe und m für Null bis 40 steht.
   Novolake der allgemeinen Formel II sind durch säurekatalysierte Reaktion von Phenol bzw. Kresol und Epoxidierung der Reaktionsprodukte erhältlich: 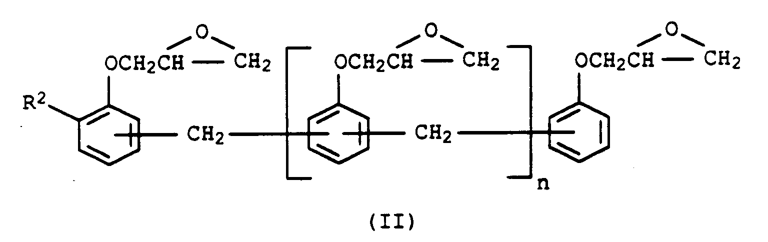 wobei R² für Wasserstoff oder eine Methylgruppe und n für 0 bis 40 steht.
- Glycidylether von Reaktionsprodukten aus Phenol und einem Aldehyd
   Durch säurekatalysierte Umsetzung von Phenol und Aldehyden und anschließende Epoxidierung mit Epichlorhydrin sind Glycidylether zugänglich, z.B. ist 1,1,2,2-Tetrakis-[4-(2,3-epoxypropoxy)phenyl]ethan aus Phenol und Glyoxal zugänglich.
- Aromatische Glycidylamine
   Beispielhaft sind Triglycidylverbindung von p-Aminophenol, 1-(2,3-epoxypropoxy)-4-[N,N-bis(2,3-epoxypropyl)amino]benzol, und die Tetraglycidylverbindung von Methylendiamin Bis{-4-[N,N-bis(2,3-epoxypropyl)amino]phenyl}methan zu nennen.

Im einzelnen sind weiterhin zu nennen:
1,1,2,2-Tetrakis[4-(2,3-epoxypropoxy)phenyl]ethan, Tris[4-(2,3-epoxypropoxy)phenyl]methan-isomere, 2,5-Bis[(2,3-epoxypropoxy)phenyl]octahydro-4,7-methano-5H-inden.

Bevorzugte Ausgangsverbindungen sind Di-[p-glycidoxi-phenyl]-methan und 2,2-Di-[p-glycidoxi-phenyl]-propan und Oligomere dieser Verbindungen.

Als Katalysatoren für das erfindungsgemäße Verfahren kommen homogene Rutheniumkatalysatoren in Betracht. Diese werden durch Reduktion von Rutheniumverbindungen erhalten. Als Rutheniumverbindungen sind insbesondere Ru-III-Verbindungen wie Rutheniumtrichlorid und Rutheniumacetylacetonat zu nennen.

Die genannten Rutheniumverbindungen können mit einer Reihe von Reduktionsmitteln zu homogenen Katalysatoren umgesetzt werden. Im einzelnen kommen in Betracht:
- Metalle mit einem Redoxpotential von -0,75 bis -2,5 V wie Mg, Al, Zn und Na, die bevorzugt als Pulver verwendet werden,
- Borhydride wie Natriumborhydrid,
- Aluminiumhydride wie Dialkylaluminiumhydride, z.B. Di-butyl-aluminiumhydrid, und Alanate wie Lithiumaluminiumhydrid,
- Aluminiumalkylverbindungen wie Triethylaluminium,
- Lithiumalkylverbindungen wie Butyllithium und Lithiumarylverbindungen wie Phenyllithium.

Die Reduktionsmittel werden mit der Rutheniumverbindung umgesetzt, wobei in der Regel Temperaturen von 20 bis 100°C für 0,5 bis 20 Stunden herrschen. Dabei werden im allgemeinen mindestens 1 Reduktionsäquivalent der genannten Verbindungen pro Äquivalent der Rutheniumverbindung eingesetzt, es können aber auch große Überschüsse an Reduktionsmittel eingesetzt werden.

Die Reduktion wird in Gegenwart eines Ethers vorgenommen. Es kommen insbesondere aliphatische C₃-C₃₀-Ether wie Tetrahydrofuran, Dioxan, Glykoldimethylether, Formaldehyddimethylacetal, Acetaldehyddimethylacetal, Diethylether und Methoxypropanol in Betracht. Durch Komplexierung der reduzierten Rutheniumverbindungen stabilisieren diese Ether den homogenen Katalysator. Die Menge an Ether kann 30 bis über 99 Gew.-%, bezogen auf die eingesetzte Rutheniumverbindung, betragen.

In einer bevorzugten Ausführungsform wird die Reduktion der Rutheniumverbindung in Gegenwart der zu hydrierenden Ausgangsverbindung vorgenommen, so daß die Hydrierung ohne Umfüllen einzelner Komponenten vorgenommen werden kann.

Die Hydrierung wird in an sich bekannter Weise vorgenommen. Die Ausgangsverbindung wird dazu mit dem Katalysator - sofern dieser nicht schon in Gegenwart der Ausgangsverbindung hergestellt wurde - und gegebenenfalls mit einem Lösungsmittel versetzt. Als Lösungsmittel kommen bevorzugt die Ether in Betracht, in deren Gegenwart die Reduktion der Rutheniumverbindung erfolgt. Die Mengen betragen im allgemeinen 5 bis 90 Gew.-%, bezogen auf den Reaktionsansatz. Die Hydrierung erfolgt mit Wasserstoff, wobei der Druck in der Regel 80 bis 320 bar, bevorzugt 200 bis 310 bar beträgt. Die Reaktionstemperatur liegt im allgemeinen bei 30 bis 80°C, bevorzugt bei 40 bis 70°C. Die Reaktion ist im allgemeinen nach 2 bis 10 Stunden beendet. Der Reaktionsansatz kann dann zur Aufarbeitung auf Normaldruck entspannt werden, von während der Reaktion ausgefallenen Feststoffen getrennt und durch Destillation von allen flüchtigen Bestandteilen befreit werden.

Das erfindungsgemäße Verfahren erlaubt eine selektive Hydrierung aromatischer Gruppen in Gegenwart von Epoxygruppen. Die dabei verwendeten Katalysatoren können leicht im Reaktionsgemisch hergestellt werden. Die zur Herstellung der Katalysatoren erforderlichen Rutheniumverbindungen können auch in kleinen Mengen genau und einfach dosiert werden.

Die Verfahrensprodukte finden als lichtbeständige Anstrichmittel, Gießharze und Laminate Verwendung.

### Beispiele

### Beispiel 1

24,4 g Rutheniumtrichloridhydrat (Gehalt an Ruthenium: 10 g) wurden in 1000 g Tetrahydrofuran unter Stickstoff mit 75 g Magnesiumpulver 5 h unter Rückfluß erhitzt und anschließend filtriert.

33,2 g der so erhaltenen Lösung wurden in einem Autoklaven mit 75 g eines Bisglycidylethers eines Phenol-Formaldehyd-Kondensates (Bis[4-(2,3-epoxypropoxy)phenyl]methan mit einem Epoxidäquivalentwert von 168) und 50 g Tetrahydrofuran bei 250 bar ansteigend von 50 auf 70°C 8 h hydriert. Nach Filtration und Abdestillieren der flüchtigen Bestandteile verblieben 76 g eines farblosen Epoxidharzes, dessen Aromatenanteile zu 93,5 % hydriert waren (durch ¹H-NMR-Spektroskopie bestimmt). Das Epoxidäquivalentgewicht (bestimmt nach ASTM D 1652-88) betrug 184.

### Beispiel 2

2,48 g Rutheniumtrichloridhydrat (1,02 g berechnet als Ruthenium), 7,5 g Magnesiumpulver, 1 kg des in Beispiel 1 charakterisierten Bisglycidylethers und 1 kg Tetrahydrofuran wurden 2 h bei 70°C unter Stickstoff gerührt. Anschließend wurde 14 h bei 250 bar und 50 bis 70°C hydriert. Nach Aufarbeitung wie in Beispiel 1 wurden 1030 g Epoxyharz isoliert (Aromatenhydrierung: 93,3 %, Epoxidäquivalentgewicht 177).

### Beispiel 3

In Analogie zu Beispiel 2 wurden 4,5 g Aluminium- statt Magnesiumpulver eingesetzt. Ausbeute: 1032 g (Aromatenhydrierung 100 %, Epoxidäquivalentgewicht 181).

### Beispiel 4

183 mg Rutheniumtrichloridhydrat in 60 g Tetrahydrofuran wurden bei -50°C mit 4,5 ml einer 20 gew.-%igen Lösung von Dibutylaluminiumhydrid in Hexan versetzt. Bei Raumtemperatur wurden 75 g des in Beispiel 1 charakterisierten Bisglycidylethers zugesetzt und bei 250 bar und 50 bis 70°C hydriert.

Nach Zugabe von 1 g Wasser (zur Hydrolyse überschüssigen Hydrids) und 1 g Aktivkohle wurde der Reaktionsaustrag filtriert und die flüchtigen Bestandteile wurden abdestilliert. Man erhielt 73 g eines leicht gefärbten Epoxyharzes (Aromatenhydrierung: 88,2 %, Epoxidäquivalentgewicht 179).

### Beispiel 5

490 mg Rutheniumtrisacetylacetonat (100 mg berechnet als Ruthenium) wurden in 20 ml THF bei 100°C durch Zugabe von 510 mg Triethylaluminium (2,5 ml einer 25 %igen Lösung in Toluol) reduziert.

Anschließend wurden 100 g des in Beispiel 1 beschriebenen Bisglycidylethers und 80 ml THF zugegeben und die erhaltene Lösung 5 h bei 70°C und einem Wasserstoffdruck von 250 bar umgesetzt.

Nach einer Aufarbeitung wie in Beispiel 4 beschrieben erhielt man 103 g eines leicht gefärbten Epoxyharzes (Aromatenhydrierung 91,3 %, Epoxidäquivalentgewicht 181).

### Beispiel 6 (Reduktion in Gegenwart eines Glycidylethers)

2,5 g Rutheniumtrichloridhydrat (1,02 g Ruthenium), 2,5 g Magnesium, 1 kg des in Beispiel 1 charakterisierten Bisglycidylethers und 1 kg THF wurden 6 h bei 70°C und 250 bar Wasserstoffdruck umgesetzt. Nach Filtration und Abdestillieren flüchtiger Komponenten wurden 1037 g eines farblosen Epoxyharzes (quantitative Aromatenhydrierung, Epoxidäquivalentgewicht 178) isoliert.

## Patentansprüche

1. Verfahren zur selektiven Hydrierung von aromatischen Gruppen organischer Moleküle, die mindestens eine aromatische Gruppe und eine Epoxygruppe tragen, mit Wasserstoff in Gegenwart eines rutheniumhaltigen Katalysators, dadurch gekennzeichnet, daß man einen homogenen Rutheniumkatalysator verwendet, der durch Reduktion einer Rutheniumverbindung mit einem Metall, das ein Redoxpotential von -0,75 bis -2,5 V besitzt, einem Borhydrid, einem Aluminiumhydrid, einer Aluminiumalkylverbindung, einer Lithiumalkyl oder -arylverbindung in Gegenwart eines Ethers erhältlich ist.

2. Verfahren nach Anspruch 1, dadurch gekennzeichnet, daß man Di-[p-glycidoxi-phenyl]methan oder 2,2-Di-[p-glycidoxi-phenyl]-propan hydriert.

3. Verfahren nach Anspruch 1 oder 2, dadurch gekennzeichnet, daß man die Reduktion der Rutheniumverbindung mit Magnesium oder Aluminium vornimmt.

4. Verfahren nach den Ansprüchen 1 bis 3, dadurch gekennzeichnet, daß man Rutheniumtrichlorid reduziert.

5. Verfahren nach Anspruch 1 bis 4, dadurch gekennzeichnet, daß man die Reduktion der Rutheniumverbindung in Gegenwart der zu hydrierenden organischen Verbindung vornimmt.

6. Verfahren nach den Ansprüchen 1 bis 5, dadurch gekennzeichnet, daß man die Reduktion der Rutheniumverbindung in Gegenwart von Tetrahydrofuran, Dioxan, Glykoldimethylether, Diethylether oder Methoxypropanol vornimmt.

## Claims

1. A process for the selective hydrogenation of aromatic groups in organic molecules having at least one aromatic group and one epoxy group with hydrogen in the presence of a ruthenium-containing catalyst, wherein a homogeneous ruthenium catalyst which is obtainable by reducing a ruthenium compound with a metal which has a redox potential of from -0.75 to -2.5 V, with a borohydride, with an aluminum hydride, with an alkylaluminum compound, or with an alkyl- or aryllithium compound in the presence of an ether, is used.

2. A process as claimed in claim 1, wherein di[p-glycidoxyphenyl]methane or 2,2-di[p-glycidoxyphenyl]propane is hydrogenated.

3. A process as claimed in claim 1 or 2, wherein the reduction of the ruthenium compound is carried out with magnesium or aluminum.

4. A process as claimed in claims 1 to 3, wherein ruthenium trichloride is reduced.

5. A process as claimed in claims 1 to 4, wherein the reduction of the ruthenium compound is carried out in the presence of the organic compound to be hydrogenated.

6. A process as claimed in claims 1 to 5, wherein the reduction of the ruthenium compound is carried out in the presence of tetrahydrofuran, dioxane, glycol dimethyl ether, diethyl ether or methoxypropanol.

## Revendications

1. Procédé d'hydrogénation sélective des groupements aromatiques de molécules organiques portant au moins un groupement aromatique et un groupement époxy, avec de l'hydrogène en présence d'un catalyseur contenant du ruthénium, caractérisé en ce que l'on utilise un catalyseur homogène au ruthénium pouvant être obtenu par réduction d'un composé du ruthénium avec un métal possédant un potentiel rédox de -0,75 à 2,5V, un hydrure de bore, un hydrure d'aluminium, un composé alkylé de l'aluminium, un composé alkylé ou arylé du lithium en présence d'un éther.

2. Procédé selon la revendication 1, caractérisé en ce que l'on hydrogène le di[p-glycidoxyphényl]méthane ou le 2,2-di[p-glycidoxyphényl]propane.

3. Procédé selon la revendication 1 ou 2, caractérisé en ce que l'on effectue la réduction du composé du ruthénium avec du magnésium ou de l'aluminium.

4. Procédé selon l'une quelconque des revendications 1 à 3, caractérisé en ce que l'on réduit du trichlorure de ruthénium.

5. Procédé selon l'une quelconque des revendications 1 à 4, caractérisé en ce que l'on effectue la réduction du composé du ruthénium en présence du composé organique à hydrogéner.

6. Procédé selon l'une quelconque des revendications 1 à 5, caractérisé en ce que l'on effectue la réduction du composé du ruthénium en présence de tétrahydrofurane, de dioxane, de glycoldiméthyléther, de diéthyléther ou de méthoxypropanol.
